# EUROPEAN PATENT APPLICATION

(11) **EP 1 818 403 A1**
(43) Date of publication of application: **15.08.2007**
(21) Application number: 05857623.2
(22) Date of filing: 11.08.2005
(51) Int. Cl.: C12N 15/47, A61K 35/76, A61K 39/205, A61P 31/14

(54) **MUTANT G GENES OF THE TROUT VIRAL HAEMORRHAGIC SEPTICAEMIA VIRUS (VHSV) AND APPLICATIONS THEREOF**

(30) Priority: 27.08.2004 ES 200402092
(71) Applicant: Instituto Nacional De Investigacion y Tecnologia agraria y Alimentaria (INIA), 28040 Madrid (ES)
(72) Inventor: COLL MORALES, Julio, E-28040 Madrid (ES)
(74) Representative: Gonzalez Palmero, Fé
(86) International application number: PCT/ES2005/000459
(87) International publication number: WO 2006/035082

(57) **Abstract**

The invention relates to mutant G genes of viral haemorrhagic septicaemia virus (VHSV) in rainbow trout, which encode mutant G proteins of VHSV with defective or null binding to cells that can be infected by VHSV. Said mutant G genes of VHSV can be used, among other applications, to produce vaccines (DNA vaccines or attenuated live vaccines) for preventing disease caused by VHSV in animals that can be infected by VHSV, generating non-human transgenic animals, and developing reagents for the diagnosis of infection caused by VHSV.

## Description

### FIELD OF THE INVENTION

The invention relates, in general, to mutant G genes of haemorrhagic septicaemia virus (VHSV) in rainbow trout, which encode mutant G proteins of VHSV with defective or null binding to cells that can be infected by VHSV. Said mutant G genes of VHSV can be used, among other applications, to produce a vaccine for preventing the disease caused by VHSV in animals that can be infected by VHSV.

### BACKGROUND OF THE INVENTION

Rhabdoviruses are one of the major causes of death in fish farms, causing great losses in the salmon breeding industry. Among the rhabdoviruses that affect fish (novirhabdoviruses), viral haemorrhagic septicaemia virus (VHSV), which originated in Europe but has recently spread to America, is one of the most dangerous, as it not only affects salmonids but also cod, turbot, croaker, eels, John Dory and prawns. Despite many efforts, including successful DNA vaccines at laboratory level, a commercial vaccine against VHSV is not yet available.

VHSV is a rhabdovirus whose viral particle, measuring approximately 170 x 80 nm, consists of an inner nucleocapsid that surrounds a negative-sense single-stranded RNA molecule [ssRNA(-)] with 11,000 bases and a molecular weight of 5-6.4 x 10⁶ kDa, and a bullet-shaped outer shell consisting of a lipoprotein membrane and outwardly-projecting trimeric spikes. The complete VHSV genome has already been sequenced (Heike et al., 1999). The virus comprises L, G, N M1 and M2 proteins. The L protein (190 kDa), which is associated with viral RNA, presents transcriptase and replicase activity. The G protein or pG (65 kDa) is a glycoprotein that forms trimeric spikes that are responsible for producing neutralising antibodies (Ab). The N nucleoprotein (40 kDa) of the nucleocapsid is the major protein. An Nx protein has also been described in VHSV, which is antigenically related to the N protein and whose function is unknown. The M1 or P phosphoprotein (19 kDa) is associated with the L polymerase protein. The M2 or M protein (25 kDa) can either be situated around the lipid membrane or inside the nucleocapsid. The infection caused by rhabdovirus begins when the virus binds, by means of the pG, to specific receptors in the outer membrane of the host, followed by membrane fusion dependent on a reduction in pH after the virus has entered the cytoplasm of the cell by endocytosis. Once inside the cells, the rhabdovirus replicates in the cytoplasm, the virions mature and, finally, they bud from the cell surface, lysing the cell.

Mutant pGs of vesicular stomatitis virus (VSV), a very well-studied rhabdovirus in mammals, have been described with mutations located in both the fusion peptide and in the carboxy-terminal regions that affect the conformational changes at low pHs required for viral fusion. The alignment of the pG of VSV with the pGs of another 14 animal rhabdoviruses has made it possible to predict the locations of the hypothetical fusion peptides in other rhabdoviruses, including VHSV (Walter & Kongsuwan, 1999). According to this model, the fusion peptide of VHSV could be located between positions 142 and 159 of the pG of VHSV.

Indirect evidence achieved using recombinant and synthetic peptides of the VHSV pG seems to suggest that the sequence between amino acids 56 and 110 (frg11) containing non-canonical heptad repeats and the phospholipid (p2) binding peptide, could be involved in fusion. When recombinant frg11 was added to a cell monolayer dramatic changes were observed in said recombinant frg11, in terms of both its solubility and the beta sheet conformation at low pH, as well as inducing low-pH-dependent cell-cell fusion. Some mutant forms of the pG of VHSV (118-161) obtained by resistance to neutralisation by monoclonal antibody (MAb) C10 retained viral fusion capacity, despite having alterations in its conformation (Gaudin et al, 1999). MAb C10 or anti-frg11, anti-p2 (82-109) and anti-p4 (123-144) antibodies (Fredericksen et al, 1999) inhibit viral fusion, which suggests that those regions may be involved in viral fusion to the host animal cell. Due to the presence of a disulphide bridge between positions 110 and 152 (Einer-Jensen et al, 1998), it is thought that p2 and the fusion peptide must occupy nearby positions in the native pG of VHSV. However, as yet there is no direct evidence for the involvement of these regions in viral fusion to the host animal cell.

Due to the significant incidence of rhabdovirus infections in fish, VHSV in particular, and the lack of available commercial vaccines, there is a need to develop effective vaccines against VHSV and other rhabdoviruses.

### SUMMARY OF THE INVENTION

It has now been possible to express mutant G genes of VHSV with mutations in the p2 and hypothetical fusion peptide regions, and in the regions between them, in the membrane of a fish cell line, and reactivity assays have been carried out with conformation-dependent monoclonal antibodies (MAbs), including monoclonal antibody (MAb) C10 and cell-cell fusion assays at different pHs. This study has made it possible to identify four mutations (P79A, L85S, R103A and T135E) that, despite not reacting with said MAbs, retain some of the fusion activity similar to that of the MAb C10-resistant mutants. Three of these mutations (P79A, L85S and T135E), which are mapped around two specific pG locations (80 and 140), show amino acid variations between different VHSV isolates. Due to the fact that 40% of the VHSV-immunised rainbow trout strongly recognised linear epitopes in these regions, the mutant G genes provided by this invention can potentially be used to design vaccines for protecting animals from infection caused by VHSV. Said vaccines may be, for example, DNA vaccines or attenuated live vaccines.

By obtaining mutants that affect the early stages of VHSV infection, it is possible to develop therapeutic methods and/or vaccines for VHSV and other rhabdoviruses. Knowing about the mechanism of processes such as fusion makes it possible to develop chemical products that could be used to interfere in the process, becoming therapeutic products, i.e. products that could be used to check the spread of the disease once the epizooty is developed. Moreover, knowing more about the VHSV sequences involved in processes such as fusion makes it possible to design multiple mutants that result in the attenuation of the virus and can be used to develop attenuated live vaccines or a DNA vaccine.

Therefore, one aspect of the invention relates to a mutant G gene of VHSV that encodes a mutant pG of VHSV, comprising at least one mutation with defective or null binding to cells that can be infected by VHSV.

Another aspect of the invention relates to a vector comprising said mutant G gene of the invention and its use in producing a vaccine for protecting animals that can be infected by VHSV. Host cells comprising said vector are an additional aspect of this invention.

Another aspect of the invention relates to a mutant VHSV whose genome comprises said mutant G gene of the invention, together with the other VHSV genes. The use of said mutant VHSV in producing a vaccine for protecting animals that can be infected by VHSV is an additional aspect of this invention. Host cells transfected or infected with said mutant VHSV are also an additional aspect of this invention.

Another aspect of the invention relates to a vaccine comprising the mutant G gene of the invention, and, optionally, one or more adjuvants and/or pharmaceutically acceptable vehicles. Said mutant G gene can be incorporated into said vector or into said VHSV. In a particular embodiment, said vaccine is selected from a DNA vaccine and an attenuated live vaccine.

Another aspect of the invention relates to a transgenic non-human animal whose cells contain a mutant G gene of the invention integrated into their genome.

Another aspect of the invention relates to a mutant pG of VHSV encoded by said mutant G gene of the invention. The procedure for producing said mutant pG is an additional aspect of this invention.

### BRIEF DESCRIPTION OF THE FIGURES

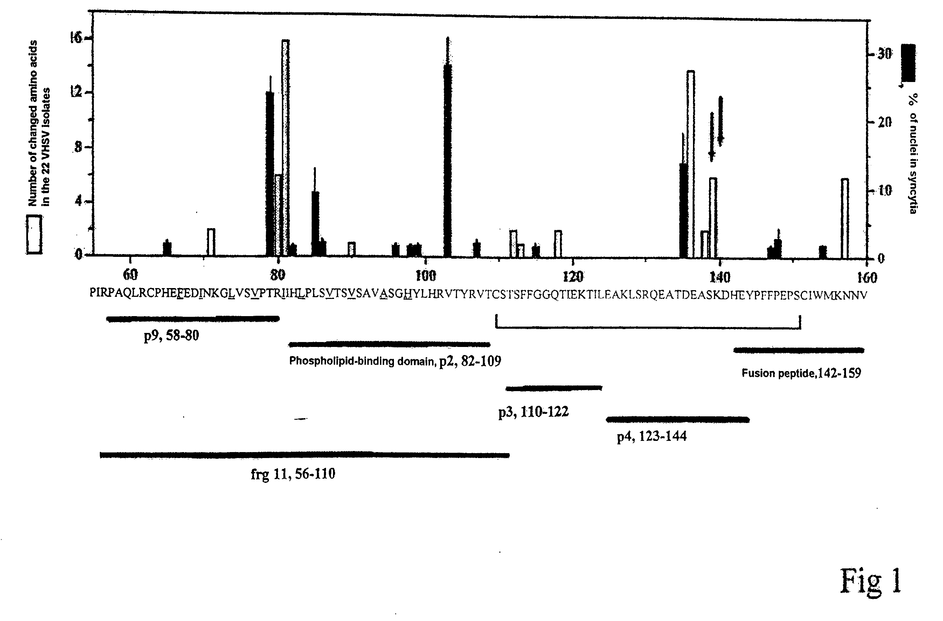

- Figure 1: shows the location, the number of changed amino acids in the 22 VHSV isolates and the percentage of nuclei in syncytia of mutants in the corresponding region from amino acids 56 to 159 of the G protein of VHSV. Cysteines are shown in bold. The disulphide bridges between C110 and C152 are shown as a horizontal line connecting the cysteines (Einer-Jensen et al., 1998). The positions and locations of p9, p2 (phospholipid-binding domain), p3 (cold water fish rhabdovirus conserved sequence), p4 (hydrophilic loop) and frg11 (p9+p2) sequences in pG [as defined in previous work (Estepa et al., 2001)] and the hypothetical fusion peptide (Walter & Kongsuwan, 1999) are indicated by thick horizontal lines. The vertical arrows indicate the locations of the MAb C10-resistant mutants in positions 139 and 140 that did not lose fusion (Gaudin et al, 1999). Hydrophobic heptad repeat amino acids are underlined (Coll, 1995).

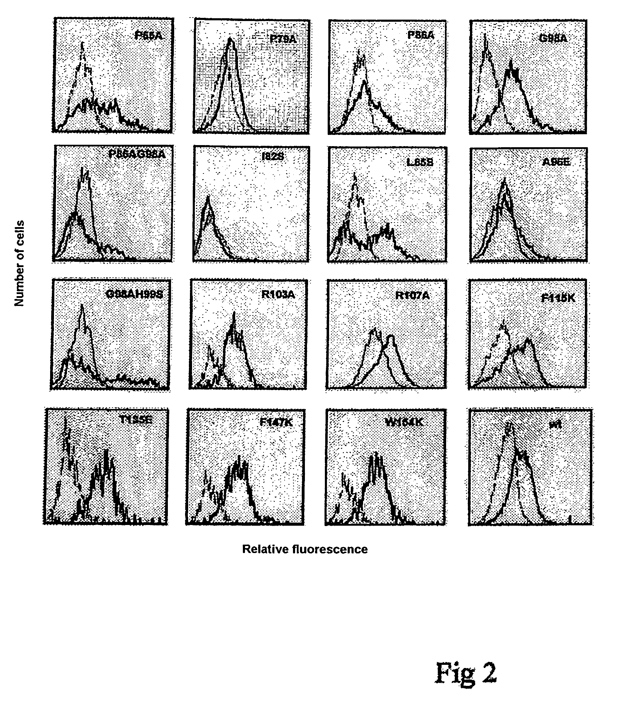

- Figure 2: shows the representative FACS profiles obtained by staining EPC cells transfected with the different pGs of mutated VHSV and EPC cells not transfected with anti-pG polyclonal antibodies (PAbs). EPC cell monolayers were transfected with the pMCV1.4 plasmids that encoded for each of the mutant pGs of VHSV (mutant pMCV1.4-pG). Non-transfected EPC cell monolayers were prepared in parallel. Two days later, both the transfected EPC cells and the non-transfected cells were stained with anti-pG PAbs and FITC-GAR. The cells were separated from the monolayers and analysed by FACS. The experiments were repeated 2-6 times for each mutant. A representative experiment is shown in the figure, whilst the mean and standard deviation are shown in Table 1. The P148K mutant was omitted from the figure to improve the presentation. Relative fluorescence is in logarithmic units. Non-transfected EPC cells are shown in grey and transfected EPC cells are shown in black.

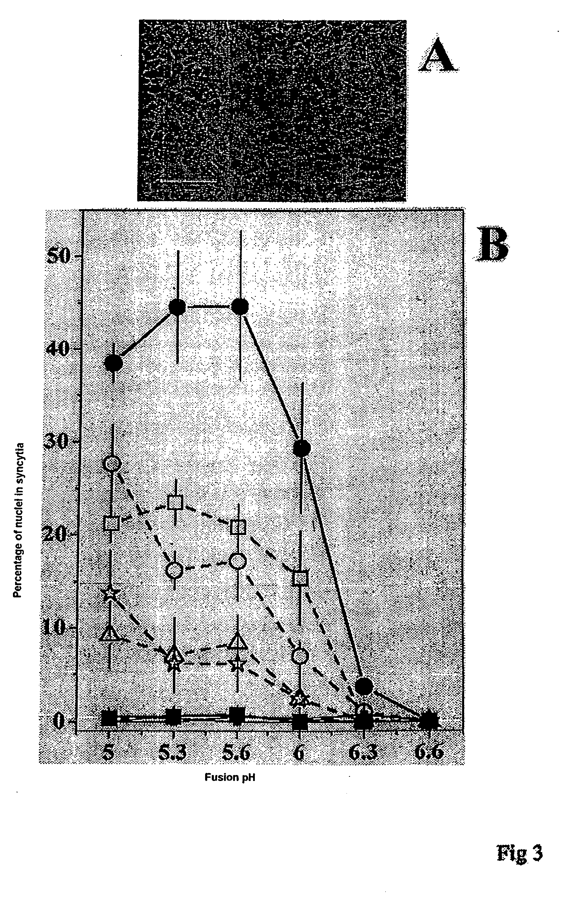

- Figure 3: shows the appearance of syncytia (A) and the percentage of nuclei in syncytia induced by low pH in EPC cells transfected with pMCV1.4 plasmids that encoded for the mutant pGs of VHSV (mutant pMCV1.4-pG) (B). EPC cell monolayers were transfected with the pMCV1.4 plasmids that encoded for each mutant pG of VHSV. Two days later, the cell culture medium was replaced by medium at different pHs for 15 minutes and then by fresh medium at pH 7.4 for 2 hours. The monolayers were fixed, stained and the nuclei in syncytia were counted (n=1,300). Figure 3B shows the average values from 2-3 experiments per mutant. • wild type; □, mutant P79A; O mutant R103A; Δ, mutant L85S; * mutant T135E; ■ mutant P86A, P65A, P86AG98A, R107A, F115K, F147K, W154K, P148K, A96E.

### DETAILED DESCRIPTION OF THE INVENTION

The invention relates, in general, to mutants of the G gene that encodes mutant G proteins of VHSV, comprising at least one mutation, wherein said mutation(s) that affect(s) said pG mutants produce(s) a complete or partial defect in their binding to cells that can be infected by VHSV.

Therefore, one aspect of the invention relates to a mutant G gene of VHSV that encodes a mutant pG of VHSV, hereinafter mutant G gene of the invention, wherein said mutant pG of VHSV comprises at least one mutation with defective or null binding to cells that can be infected by VHSV.

As used herein, the expression "defective binding to cells that can be infected by VHSV" means that the mutant pG of VHSV encoded by said mutant G gene of the invention has less capacity for binding to cells that can be infected by VHSV than the native pG of VHSV taken as a reference. Similarly, the expression "null binding to cells that can be infected by VHSV", as used herein, means that the mutant pG of VHSV encoded by said mutant G gene of the invention is completely incapable of binding to cells that can be infected by VHSV. The binding to a mutant G protein of VHSV can be determined by an EPC cell-cell fusion assay where the cells are transfected with a vector that comprises the mutant G gene under investigation (that encodes the mutant G protein of VHSV to be assayed), as described in Example 1, in the Materials and Methods section.

The amino acid sequence of the wild type (wt) native pG of VHSV taken here as a reference is that described by Thiry in the 07.71 strain of VHSV (Thiry, 1991). The mutant G gene of the invention is based on said nucleotide sequence of the G gene of VHSV (wt) and it includes one or more mutations in different regions of said nucleotide sequence so that it encodes a mutant pG of VHSV that comprises at least one mutation in its amino acid sequence compared to the native pG and it has defective or null binding to cells that can be infected by VHSV.

In the meaning used herein, the term "mutation" refers to the alteration of one or more nucleotides in the G gene of VHSV wt leading to a change in at least one amino acid in the native pG of VHSV as a result of expression of the nucleotide sequence where said alteration has occurred. In a particular embodiment, said mutant pG of VHSV comprises a single mutation, whilst, in another particular embodiment, said mutant pG of VHSV comprises two or more mutations.

Similarly, in a particular embodiment, the mutant G gene of the invention encodes a mutant pG of VHSV that comprises at least one mutation in its amino acid sequence compared to the native pG with defective binding to cells that can be infected by VHSV. In another preferred embodiment, the mutant G gene of the invention encodes a mutant pG of VHSV that comprises at least one mutation in its amino acid sequence compared to the native pG with null binding to cells that can be infected by VHSV.

The mutant G gene of the invention comprises one or more mutations in the nucleotide sequence of the G gene of VHSV wt that result in one or more mutations (amino acid changes or substitutions) in the amino acid sequence of the native pG, therefore, giving rise to a mutant pG with the particular characteristic that it is defective or null in terms of its fusion capacity, i.e. it is partially or completely incapable of binding to the VHSV native pG receptors in cells that can be infected by VHSV. Said mutations affect different domains of the pG of VHSV, such as the domain upstream of the p2 region, the phospholipid (p2) binding domain, the domain downstream of the p2 region and the fusion peptide domain.

For the sake of simplicity, the different mutant G genes of VHSV as exemplified herein will be identified by specifying the resulting mutation at an amino acid level. The recommendations provided in the following references have been used for the amino acid mutation nomenclature: (i) den Dunnen JT, Antonarakis SE. 2000. Mutation nomenclature extensions and suggestions to describe complex mutations: a discussion. Hum Mutat. 15(1):7-12; and (ii) Antonarakis SE. Recommendations for a nomenclature system for human gene mutations. Nomenclature Working Group. Hum Mutat. 11(1): 1-3. For example, a mutation identified as "P65A" indicates that the proline situated at position 65 of the amino acid sequence of the native pG of VHSV has been replaced by alanine.

In a particular embodiment, the mutant G gene of the invention encodes a mutant pG of VHSV whose mutation is located upstream of the p2 domain, i.e. from amino acid 58 to 80 of the amino acid sequence of the native pG of VHSV. Illustrative examples of this type of mutations include mutations P65A and P79A.

In another particular embodiment, the mutant G gene of the invention encodes a mutant pG of VHSV whose mutation is located in the phospholipid (p2) binding domain of the pG of VHSV, i.e. from amino acid 82 to 109 of the amino acid sequence of the native pG of VHSV. Illustrative examples of this type of mutations include mutations I82S, L85S, P86A, P86AG98A, A96E, G98A, G98AH99S, R103A and R107A.

In another particular embodiment, the mutant G gene of the invention encodes a mutant pG of VHSV whose mutation is located downstream of the p2 domain, i.e. from amino acid 110 to 144 of the amino acid sequence of the native pG of VHSV. Illustrative examples of this type of mutations include mutations F115K and T135E.

In another particular embodiment, the mutant G gene of the invention encodes a mutant pG of VHSV whose mutation is located in the fusion peptide domain of the pG of VHSV, i.e. from amino acid 142 to 159 of the amino acid sequence of the native pG of VHSV. Illustrative examples of this type of mutations include mutations F147K, P148K and W154K.

Surprisingly, when said substitutions are introduced to said domains of the native pG of VHSV, pG mutants of VHSV are obtained with defective or null binding to cells that can be infected by VHSV, i.e. that have partially or completely lost their capacity to bind to cells that can be infected by VHSV.

As has been mentioned above, the pG of VHSV is the viral protein that is responsible for binding the virus to cells that can be infected by VHSV and the viral protein that is responsible for the immune response of potential VHSV hosts (animals that can be infected by VHSV), which means that the mutant G gene of the invention can be used to immunise animals that can be infected by VHSV using a single gene of said virus, such as the mutant G gene of the invention, as an immunogen.

As used herein, the expression "VHSV hosts" or "animals that can be infected by VHSV" refers to any animal that can be infected by VHSV and includes aquatic animals, e.g. salmonids, such as rainbow trout, different species of salmon etc. and other aquatic animals that can be infected by VHSV such as cod, turbot, sea bass, eels, flatfish and shrimps.

The mutant G gene of the invention offers numerous applications. For example, the mutant G gene of the invention, in the absence of all or some of the other genes of the VHSV genome, and optionally incorporated into appropriate vectors, can, in itself, be used in several possible applications, e.g. (i) for producing DNA vaccines, (ii) for producing attenuated live vaccines comprising complete mutant VHSV containing a mutant G gene of the invention and the other VHSV genes, (iii) for creating transgenic non-human animals, (iv) for producing reagents for diagnosing the infection caused by VHSV, etc.

Therefore, another aspect of the invention relates to a vector, hereinafter the vector of the invention, which comprises a mutant G gene of the invention. In a particular embodiment, said vector of the invention is a plasmid DNA or an expression vector that can be expressed in eukaryotic cells, e.g. in animal cells, which comprises said mutant G gene of the invention. The vector of the invention can also contain the necessary elements for expression and translation of the mutant G gene of the invention and the elements that regulate its transcription and/or translation. When the vector of the invention is introduced, by any conventional method, e.g. by injection, transfection or transformation, in the cells of a host that can be infected by VHSV, the cells in which said vector of the invention has been introduced express a mutant pG of VHSV with defective or null, and preferably null, binding to cells that can be infected by VHSV, but that is capable of immunising the host against VHSV, as said mutant pG is directed at the cell membrane, triggering immune response reactions in the animal that are very similar to those produced by infection with the complete VHSV. It is therefore possible to vaccinate against VHSV without using the complete virus, but simply using the mutant G gene of the invention incorporated into a suitable vector (Fernández-Alonso et al., 1999; Fernández-Alonso et al., 2000). The vector of the invention, which comprises a mutant G gene of the invention, is therefore the base for a DNA vaccine and can be used in the absence of the complete virus to immunise animals that can be infected by VHSV.

Therefore, another aspect of the invention relates to the use of a vector of the invention to produce a vaccine for protecting animals that can be infected by VHSV. In a particular embodiment, said vaccine is a DNA vaccine.

Furthermore, the mutant G gene of the invention can be used to obtain a complete mutant VHSV, i.e. a VHSV containing a mutant G gene of the invention and the other VHSV genes, which can be native or mutant, independently of one another.

Therefore, another aspect of the invention relates to a possible mutant VHSV, hereinafter the mutant VHSV of the invention, whose genome comprises a mutant G gene of the invention and the other VHSV genes, wherein said mutant G gene encodes a mutant pG of VHSV, said mutant pG of VHSV comprising at least one mutation and said mutant pG of VHSV having defective binding to cells that can be infected by VHSV. The other genes that make up the mutant VHSV of the invention, which are different to the mutant G gene of the invention, can be native or mutant, independently of one another.

The mutant VHSV of the invention has defective binding to cells that can be infected by VHSV and it can therefore only partially carry out the function involved in the process of viral fusion to the cell membrane to be infected, meaning that it would be attenuated.

After experimental infection of the cells that can be infected by VHSV, the mutant VHSV of the invention expresses a mutant pG of VHSV with partial binding to cells that can be infected by VHSV, i.e. with less capacity to infect new host cells, as its membrane cannot bind 100% to the membrane of the host cell to be infected. However, said mutant VHSV would leave the host immunised. Therefore, said mutant VHSV of the invention can be used for therapeutic purposes, e.g. to prevent infection caused by VHSV.

Therefore, another aspect of the invention relates to the use of said mutant VHSV to produce vaccines for protecting animals that can be infected by VHSV. In a particular embodiment, said vaccines are attenuated live vaccines.

The mutant VHSV of the invention can be obtained by conventional methods known to a person skilled in the art. However, in a particular embodiment, said mutant VHSV of the invention can be obtained by reverse genetic techniques known to a person skilled in the art.

Another aspect of the invention also relates to a vaccine that comprises a therapeutically effective amount of a mutant G gene of the invention and, optionally, one or more adjuvants and/or pharmaceutically acceptable vehicles. In a particular embodiment, said mutant G gene of the invention is incorporated into a vector of the invention, whilst in another particular embodiment, said mutant G gene is incorporated into a mutant VHSV of the invention.

The vaccine provided by this invention can be used to protect animals that can be infected by VHSV.

In the meaning used herein, the expression "therapeutically effective amount" refers to the amount of mutant G gene of the invention that is calculated to produce the desired effect and, in general, it will be determined by the characteristics of the mutant G gene of the invention that is used and the immunisation effect to be achieved, among other factors.

The pharmaceutically acceptable vehicles that can be used in the formulation of a vaccine according to the present invention must be sterile and physiologically. compatible, e.g. sterile water, saline solution, aqueous buffers such as PBS, alcohols, polyols and suchlike. Said vaccine may also contain other additives, such as adjuvants, stabilisers, antioxidants, preservatives and suchlike. The available adjuvants include, but are not limited to, aluminium salts or gels, carbomers, nonionic block copolymers, tocopherols, muramyl dipeptide, oil emulsions, cytokines, etc. The amount of adjuvant that should be added depends on the nature of the adjuvant. The stabilisers available for use in vaccines according to the invention are, e.g. carbohydrates, including sorbitol, mannitol, dextrin, glucose and proteins such as albumin and casein, and buffers such as alkaline phosphatase. The available preservatives include, among others, thimerosal, merthiolate and gentamicin.

The vaccine provided by this invention can be administered by any appropriate route of administration that results in an immune response to protect against VHSV, for which said vaccine will be formulated in a manner that is suitable for the chosen route of administration. In a particular embodiment, the vaccine is formulated to be introduced into the animal when it is immersed in a dip bath containing said vaccine; in another particular embodiment, the vaccine is prepared for administration by injection. For example, said vaccine can be prepared in the form of an aqueous solution or suspension, in a pharmaceutically acceptable vehicle, such as saline solution, phosphate buffered saline (PBS), or any other pharmaceutically acceptable vehicle.

The vaccine provided by this invention can be a DNA vaccine (using a vector of the invention that comprises a mutant G gene of the invention) or an attenuated live vaccine (based on a mutant VHSV of the invention whose genome comprises a mutant G gene of the invention).

The vaccine provided by the present invention can be prepared using conventional methods known by a person skilled in the art. In a particular embodiment, said vaccine is prepared using the mixture, if applicable, of a vector of the invention or a mutant VHSV of the invention, optionally having one or more adjuvants and/or pharmaceutically acceptable vehicles.

Additionally, the mutant G gene of the invention can be used to generate a transgenic non-human animal whose cells contain a mutant G gene of the invention integrated into their genome. Said non-human animal can be any animal, such as an aquatic animal, e.g. a fish. For example, said fish could be a salmonid, such as rainbow trout. The transgenic non-human animal provided by this invention expresses a mutant pG of VHSV that comprises at least one mutation with defective or null binding to cells that can be infected by VHSV. Said transgenic non-human animals can be obtained by conventional methods known by a person skilled in the art, e.g. from a mutant G gene of the invention or from a vector that contains it, such as a vector of the invention. Information about gene transfer to eukaryotic cells and entire organisms can be found, for instance, in the book entitled "Ingeniería genética y transferencia génica", by Marta Izquierdo, Ed. Pirámide (1999), especially in Chapter 8.

The mutant G gene of the invention can also be used to produce reagents for diagnosing the infection caused by VHSV, e.g. for producing probes for genetic testing, antibodies obtained in fish, etc. Obtaining anti-VHSV antibodies for diagnosis is problematic, as they cannot be very efficiently obtained in mammals. At 37°C in the body of rabbits or mice, the virus and its proteins, especially pG, are denaturalised, making it difficult to obtain high titre anti-VHSV antibodies in mammals. Moreover, although the optimum temperature for obtaining neutralising antibodies would be 20°C e.g. in salmon or rainbow trout, is not possible with the complete virus as VHSV kills said species before antibodies are produced. The use of attenuated viruses would therefore make it possible to obtain anti-VHSV antibodies in fish at low temperatures.

The mutant G gene of the invention can be obtained by conventional methods known to a person skilled in the art. However, in a particular embodiment, said mutant G gene of the invention can be obtained by introducing the desired mutation by site-directed mutagenesis. Briefly, as described in the Materials and Methods section (Example), this is done by subjecting the pGEMTeasy-G plasmid vector containing the native pG of VHSV, under the control of the T7 promoter, to a polymerase chain reaction (PCR) using 2 primers with 15 nucleotides each that provide the desired mutation for each mutation that is to be assayed. The mutant G genes are then subcloned into pMCV1.4 plasmids to produce the corresponding pMCV1.4-G plasmids (each carrying a mutant G gene with the desired mutation) in order to carry out assays to determine expression of the mutant pG (by flow cytometry) in the surface membrane of EPC cells (epithelial cell line from carp) transfected with said pMCV1.4-G.

Although in the practical embodiment illustrated in the present invention the method used to create the mutant G gene sequences was site-directed mutagenesis, it is possible to use any other method known in the state of the art that produces the same results. Similarly, although in the present invention mutations in the genome are achieved by nucleotide substitution, it would be equally valid to use any other procedure that produces a similar result to that achieved by substitution.

Another aspect of the invention relates to a mutant pG of VHSV encoded by a mutant G gene of the invention, hereinafter the mutant pG of the invention, which is selected from a mutant pG with the P65A mutation; a mutant pG with the P79A mutation; a mutant pG with the I82S mutation; a mutant pG with the L85S mutation; a mutant pG with the P86A mutation; a mutant pG with the P86AG98A mutation; a pG mutant with the A96E mutation; a mutant pG with the G98A mutation; a mutant pG with the G98AH99S mutation; a mutant pG with the R103A mutation; a mutant pG with the R107A mutation; a mutant pG with the F11K mutation; a mutant pG with the T135E mutation; a mutant pG with the F147K mutation; a mutant pG with the P148K mutation; and a mutant pG with the W154K mutation.

Said mutant pG of the invention contains one or two mutations with defective or null binding to cells that can be infected by VHSV. For example, the mutant G protein of the invention (resulting from expression of the mutant G gene of the invention) can be completely incapable of binding to cells that can be infected by VHSV (i.e. to the receptors or cell membranes of the native G protein of VHSV), such as the mutant G proteins of VHSV identified as P65A, P86A, P86AG98A, A96E, G98A, G98AH99S, R107A, F115K, F147K, P148K and W154K (see Table 1) or it can be capable of binding to cells that can be infected by VHSV but with less binding capacity (i.e. the mutant G proteins are less capable of binding to the receptors or cell membranes of the native G protein of VHSV than the native G protein of VHSV), such as the mutant G proteins of VHSV identified as P79A, L85S, R103A and T135E, which show a receptor-binding capacity of between 9.2 ± 3.5% at pH 5.0 (L85S) and 27.7 ± 4.1% at pH 5.0 (R103A) or 23.5 ± 2.4% at pH 5.3 (P79A) [Table 1]. It has not been possible to draw conclusions about the potential defective fusion properties of the mutant G protein of the invention with the I82S mutation since, although it was expressed in the cytoplasm, it has not been possible to detect it in transfected cell membranes.

Although it was expressed in the cytoplasm, mutant I82S was not detected in the transfected cell membranes and it is therefore not possible to draw conclusions about its potential defective fusion properties (Table 1).

The vector comprising the mutant G gene of the invention can also be used to transform or transfect a suitable host cell, such as a eukaryotic cell, e.g. a cell belonging to a higher animal (mammal, fish, etc.), which is capable of expressing said mutant G gene and producing the corresponding mutant pG of VHSV. Said mutant G gene of the invention can be integrated into a chromosome of said cell or it can be present in said cell in the form of an episomal plasmid. Said host cells can be transformed and transfected using conventional methods known to a person skilled in the art. In a particular embodiment, the mutant G gene of the invention is incorporated into a vector, such as the vector of the invention, whilst in another particular embodiment, said mutant G gene of the invention is incorporated into a mutant VHSV of the invention. It is possible to use practically any host cell that can be transformed, transfected or infected by VHSV and that can allow the virus to grow. However, in a particular embodiment, said host cell is the EPC (Epithelioma Papullosum cyprisi) cell line, i.e. an epithelial cell line from carp. Said host cells containing a mutant G gene of the invention well integrated into a chromosome or as an episomal plasmid are an additional aspect of this invention.

Another aspect of the invention relates to a method for producing a mutant pG of the invention that consists of culturing a cell comprising a mutant G gene of the invention under conditions that allow the expression of said gene and, if desired, removing the mutant pG produced from the culture medium. The culture conditions will depend, among other factors, on the cell that is used. Although it is possible to use practically any host cell that can be transformed, transfected or infected by VHSV and that is capable of allowing VHSV to grow, in a particular embodiment, said host cell used to produce the mutant G protein of the invention is the EPC cell line.

The following examples illustrate the invention and must not be considered to limit its scope.

### EXAMPLE 1

### Mutant G genes of VHSV

### I. MATERIALS AND METHODS

### Plasmids used

To generate the pGEMTeasy-G construct carrying the native pG gene of VHSV, first the pcDNAI construct [provided by Dr Michel Brémont, INRA (Institute National Recherche Agronomique), Jouy en Josas, Paris, France] containing the pG gene of VHSV (French isolate 07.71) was cloned into the pcDNAI vector (4.0 kpb) (Invitrogen), and subcloned into the pcDNAI/Amp commercial vector (4.8 kpb) (Invitrogen) [Fernández-Alonso, 1999] and then into the pGEMTeasy commercial vector (Stratagene) controlled by the T7 promoter.

To carry out fluorescence-based fusion assays or syncytia formation assays, the collection of pG mutants produced by pGEMTeasy was subcloned into the pMCV1.4 plasmid (Ready Vector, Madrid, Spain). To do this, the gene of the native pG of VHSV was produced by a preparative digestion using 2 µg of the pGEMTeasy-G construct with EcoRI (10 U/µl) (GibcoBRL, Postfach, Germany) for 2 h at 37°C in Techne dri-block apparatus. Likewise, the pMCV1.4 vector was linearised. The EcoRI was inactivated at 65°C for 15 minutes and then shrimp alkaline phosphatase (SAP) (Roche, Barcelona, Spain) was added. The mixture was incubated at 37°C for 60 minutes and then the alkaline phosphatase was inactivated at 65°C for 15 minutes. The digestion products were separated in a low melting point (LMP) agarose gel at 1%, extracting and purifying the bands obtained using columns of the SNAP commercial kit (Invitrogen, Barcelona, Spain). The plasmid and the insert containing the pG-encoding sequence were ligated (plasmid: insert ratio of 1:3, including controls without the insert) at room temperature for 2 h using T4 DNA ligase (Roche) with a final volume of 20 µl per ligation mixture per mutant.

### Site-directed mutagenesis

Site-directed mutagenesis was based on the Quick-Change method (Stratagene, La Jolla, Ca, USA) for generating the mutated G genes in the pGEMTeasy-G plasmid (containing the native pG gene of VHSV) [Carneiro et al., 2001]. Two 15-nucleotide oligos containing the desired mutations were designed for each mutant. In all cases, the oligos were extended by polymerase chain reaction (PCR) using Pfu turbo DNA polymerase (Stratagene), generating unmethylated mutant plasmids in an open-chain form containing the mutation introduced in the oligos. The mixture was then treated with the specific DpnI restriction endonuclease of methylated DNA, which only digests initial parental DNA chains, the amplified plasmid remaining intact. Said plasmid, containing the desired mutation, was subsequently used to transform XL1-Blue competent cells (Stratagene). The mutated pG gene mutants were subcloned into the EcoRI site of plasmid pMCV1.4 (Rocha et al., 2004a, Rocha et al., 2004b), following the conventional methods for E. coli Top1O (Fernández-Alonso et al., 1999) to produce the corresponding pMCV1.4-G plasmids (each carrying a mutant G gene with the desired mutation). Large amounts of plasmid were prepared using the Megaprep Wizard DNA purification system (Promega, Madison, USA). The plasmid solutions were adjusted to 0.5-1 mg/ml of total DNA (absorbance at 260 nm). The mutated sequences were confirmed by sequencing the plasmids across the mutated region in both directions.

### Transfection of EPC cells with mutated plasmids

Epitelioma Papulosum cyprini (EPC) carp cells were grown [Fijan et al., 1983] on 96-well plates at 28°C with RPMI Dutch medium, HEPES buffer 20 mM and 10% of calf foetal serum (100 µl per well). The cells (approximately 100,000 cells/well) were transfected with 0.3 µg of the different pMCV1.4-G mutants previously complexed with 0.5 ml of Fugene 6 (Roche, Barcelona, Spain) (López et al., 2001; Rocha et al., 2004a; Rocha et al., 2004b) and incubated at 20°C in 5% CO₂ for two days.

### Staining of the transfected EPC cell monolayers

After transfection, the EPC cell monolayers were stained with anti-pG polyclonal antibodies (PAb) obtained in rabbits (provided by Dr Lorenzen, Denmark) [Lorenzen & LaPatra, 1999] in culture medium containing 2% rabbit serum, 2% goat serum and 2% E. coli extract for 1 hour after permeabilisation with 2-perm (BD-Biosciences, Becton-Dickinson, Spain) (to estimate the cytoplasmic expression) or without permeabilisation (to estimate the membrane expression). The cells were then incubated with the fluorescein isothiocyanate-conjugated goat anti-rabbit Fab'2 fragment (FITC-GAR) (Caltag, San Francisco, CA, USA), washed and observed under an inverted fluorescence microscope (cytoplasmic expression) or separated using FACS buffer (Becton-Dickinson) and analysed by flow cytometry (FL1 region 514-545 nm, green) in a FACScan apparatus (Becton-Dickinson) using the LYSYS II program (membrane expression). Background fluorescence profiles were achieved using non-transfected EPC cells and it was noted that they varied slightly from experiment to experiment. The following formula was used to calculate the percentage of fluorescent cells for each experiment: area under the curve obtained with transfected cells - area under the curve obtained with transfected cells overlapping with the background curve / total area under the curve obtained with transfected EPC cells x 100. To calculate the peak background fluorescence value was subtracted from the peak value obtained with transfected EPC cells. The fluorescence intensity was expressed in fluorescence relative units (fru).

### Transfected EPC cell-cell fusion assays

To carry out the fusion assays, EPC cells plated on 24-well plates (approximately 500,000 cells/well) were transfected with 0.6 mg of different pMCV1.4 mutants complexed with 2 µl of Fugene 6 (Fernández-Alonso et al., 1999; López et al, 2001; Rocha et al., 2002) and incubated at 20°C. Two days later, the transfected cell monolayers were incubated for 15 minutes in RPMI Dutch culture medium containing HEPES 20 mM / MES 20 mM (Sigma, Chem. Co., St.Louis, Missouri, USA) at different pHs (5.0, 5.3, 5.6, 6.0, 6.3, 7.0 and 7.3) at 20°C. Syncytia formation assays could not be carried out at a pH of less than 5.0 due to detachment of EPC cell monolayers. The monolayers were then incubated for 2 h at pH 7.6, fixed with cold methanol, washed, dried and stained with Giemsa (Rocha et al., 2004a; Rocha et al., 2004b). The results were expressed as the percentage of nuclei in syncytia, calculated using the following formula: number of nuclei in syncytia of three or more cells per syncytium/number of nuclei x 100.

### Phospholipid-binding assays

For the phospholipid-binding assay, 100 µl of 0.1 mg/ml of synthetic peptides (Chiron-Mimotopes, Victoria, Australia) per well (1 µg per well) were dried on 96-well plates as described above (Estepa et al., 1996a; Estepa et al., 1996b). Labelled L-3-phosphatidyl-[L-C3-¹⁹C] serine (PS) 55 mCi/mmol (Amersham, Buckinghamshire, UK) was vacuum dried in glass tubes and sonicated in 0.1 M citrate-phosphate buffer at pH 7.7 (Gaudin et al., 1993). The labelled PS was added in a volume of 100 µl per well to the solid-phase peptides (200 pmol per well). After 4 hours of incubation at 20°C, the plates were washed and extracted with 100 ml of 2% sodium dodecyl sulphate (SDS) per well in 50 µM ethylenediamine, pH 11.5 at 60°C for 30 minutes. The supernatants were pipetted onto 96-well polyethylene terephthalate plates containing 100 µl of Hiload scintillation liquid (LKB, Loughborough, UK) per well and counted in a 1450-Microbeta scintillation counter (Wallac, Turku, Finland). The background binding obtained in the absence of peptides (1.25 pmol per well) was subtracted from all the data and the counts were transformed into pmol of PS.

### II. RESULTS

### Selection of site-directed mutations

The pG sequences of 22 VHSV isolates were compared to select the mutations to introduce in the pG. The amino acid sequences corresponding to the hypothetical phospholipid-binding and fusion peptide regions (positions 56 to 159) of the 22 isolates were obtained from the GenBank (accession numbers: A10182, AB069725, AB060727, AF143862, AF345857, AF345858, AF345859, AJ233396, NC000855, U28799-2, U28747, U88056, U28800, U88050, U88051, U88052, U88053, U88054, U88055, X73873 and X66134). The translated amino acid sequences were highly conserved between the isolates. Amino acid variations between the VHSV isolates were mainly concentrated in two locations around positions 80 and 140 (Figure 1). Most of the changes were therefore found at position R81 (arginine), which changed to Q (glutamine) or K (lysine) [16 isolates], and at position D136 (aspartic), which changed to N (asparagine) [14 isolates]. Fewer amino acid variations were found in 2-4 isolates at positions 71, 80, 97, 112, 118, 138 and 139. Positions at which amino acid variations were detected were excluded from the mutant design because altered binding activity had not been noted in any of these isolates.

The positions selected for the mutation were changed to A (alanine) when possible, or to an amino acid with different physicochemical properties than those of the mutated position, depending on the possibilities for each changed nucleotide. The positions selected in the hypothetical phospholipid-binding peptide (p2+frg11) included the highly conserved helix-breaking P (proline) and G (glycine) (P65, P79, P86 and G98) and the charged arginines located in the carboxy-terminal part (R103 and R107). All these amino acids were changed to A (alanine). Other positions selected in the amino acids belonging to some of the non-canonical hydrophobic heptad repeats (I82, L85, A96) were changed to hydrophilic (S, serine) or charged (E, glutamic) amino acids. Positions F115 and T135, located between the hypothetical phospholipid-binding peptide and the fusion peptide, were mutated to a charged amino acid (K and E, respectively). As the hydrophobic amino acids F147, P 148 and W154, located in the hypothetical fusion peptide motif (F, 5Y) PXPXXCX (WF), were conserved among 14 animal rhabdoviruses (Walter & Kongsuwan, 1999), said amino acids were also mutated in VHSV to a charged amino acid (E or K).

### Expression of the mutant pG in transfected EPC cells

All plasmids containing mutant pGs obtained for VHSV were expressed in the cytoplasm of permeabilised transfected EPC cells, as verified by direct immunofluorescence with anti-G polyclonal antibodies (Table 1).

Table 1 shows that the estimated percentage of transfected EPC cells that express pG in their membranes, after taking the average of the results for 2-6 repeats per mutant, ranged from 42.4 to 77.2% (except for I82S, which had not been expressed). 53.5 ± 11% of the EPC cells transfected with the native pG gene expressed pG in their membranes. Similarly, the pG was expressed in 50.2-77.2% of the EPC cells transfected with mutants P65A, L85S, P86A, P86AG98A, G98A, G98AH99S, R103A, R107A, F115A, P148K and W154K. Mutants P79A and A96E were not transfected as efficiently as the other mutants (42.5% and 44.5%, respectively) and mutant-I82S expression in the membrane of transfected EPC cells was very low or not significantly different from the basal levels (1.3 ± 0.3%-of the transfected cells).

For each mutant, figure 2 shows a profile of non-transfected/transfected cells stained with FACS representing the 2-6 repeats indicated in Table 1. As the fusion efficiency is highly dependent on the pG density on the cell surface, the relative level of expression per cell from the FACS profiles was estimated, assuming that the antibody recognises all the mutants equally. As the background obtained with non-transfected EPC cells (grey curves on the graphs) varied slightly from one experiment to another, to compare the pG expression of the different mutants, the area overlapping with the background was removed from the fluorescence for each experiment. The average values of the intensity of FACS fluorescence were calculated from the repeats. The intensity estimated for the wild-type pG was 18.7 ± 4.1 fru (n=6) and the other mutants only varied between 10.8 and 22.5 fru, except for the I82S mutant (Table 1).

Due to the fact that, unlike the case of VSV, no proteolytic assay is available to study conformational changes induced in the pG of VHSV at low pHs, an assay of binding to conformation-dependent neutralising antibodies was used to estimate potential conformational changes induced by mutation. Correct folding of the pG was also analysed with conformation-dependent monoclonal antibodies (MAbs), such as MAb C10, which simultaneously recognises positions 140 and 433 (Bearzotti et al., 1995; Gaudin et al., 1999), and 2F1A12, which maps at position 253 (Lorenzen, personal communication). Approximately 21.6 ± 9% of the EPC cells transfected with the native form of the pG gene expressed the C10 epitope in their membranes. However, only 0.3-1.60 of the EPC cells transfected with any of the mutants expressed the C10 epitope (Table 1). Similar results were achieved with 2F1A12 monoclonal antibodies (data not shown).

### Transfected EPC cell-cell fusion assays

Figure 3 shows the typical appearance of syncytia and the fusion kinetics obtained from G gene-transfected EPC cell-cell fusion assays for the native G gene and its mutants. Under the experimental conditions used, fusion of cells transfected with the native G gene was at its maximum at pH 5.6 and it decreased to approximately 70% at pH 6.0 and to 0% at pH 6.6. Only the EPC cells transfected with mutants P79A, L85S, R103A and T135E showed any fusion activity. Mutants R103A and T135E showed a maximum fusion at pH 5.0 and the percentage of nuclei in syncytia was reduced to 27.7 ± 4.1% and 13.7 ± 4.5%, respectively. Mutants P79A and L85S showed a maximum fusion at pH 5.3-5.6 and the percentage of nuclei in syncytia was also reduced to 23.5 ± 2.4% and 9.2 ± 3.50, respectively. Mutants P79A and L85S (amino-terminal) and R103A (carboxy-terminal) flank the innermost sequences of the p2 phospholipid-binding domain.

On the other hand, mutants P86A, P86AG98A, A96E, G98A, G98AH99S and R107A (in which most of the mutations are located inside the hypothetical phospholipid-binding peptide) and P65A and F115K (in which the mutations are around the phospholipid-binding peptide) were completely defective in terms of fusion for all the pHs studied. Mutants F147A, P148A and W154A, in which the mutations are located in the highly conserved positions of the hypothetical fusion peptide, were also defective in terms of fusion for all the pHs studied (pH 5.0 or higher).

Although it was expressed in the cytoplasm, mutant I82S was not detected in the transfected cell membranes and it is therefore not possible to draw conclusions about its potential defective fusion properties (Table 1).

### Phospholipid binding of the synthetic peptides corresponding to the p2 region

As p2 (82-109) was the main region of a pepscan analysis of the pG that showed PS binding (Estepa et al., 1996a; Estepa et al., 2001), changes were introduced to a single amino acid in synthetic peptides deriving from the p2 sequence to study whether mutations in that region could affect PS binding. The amino acid sequence containing amino acids 93 to 107 (which includes the two positively charged amino acids R103 and 107) was selected to synthesise the peptides, as it showed the maximum PS-binding activity of p2 (Estepa et al., 1996a). Each amino acid in this sequence was changed to A and the effect of this change on solid-phase PS binding was measured. The PS-binding activity of the native sequence was 2.47 ± 0.34 pmol of PS per µg of peptide (Table 2). The PS-binding activity only varied from 2.1 ± 0.46 to 4.1 ± 0.53 pmol of PS per µg of peptide for the 15 synthetic peptides with changes to a single amino acid.

Due to the fact that both hydrophobic and ionic interactions are involved in PS binding by p2 (Gaudin et al., 1999), synthetic peptides were obtained in which more drastic changes were introduced at the charged amino acid positions. It was possible to change one of positions 103 or 107 to K without PS binding varying significantly (2.5 ± 0.42 or 2.6 ± 0.33 pmol of PS per µg of peptide, respectively). PS binding could only be reduced when both amino acids were simultaneously changed to K or E (1.3 ± 0.19 or 0.75 ± 0.36 pmol of PS per µg of peptide, respectively).

The substitution of several amino acids for a series of A at positions 104-106, 95 + 104-106 and 99-102 + 104-106 also reduced PS binding to 2.1 ± 0.28, 1.69 ± 0.29 and 0.69 ± 0.21 pmol of PS per µg of peptide, respectively.

### III. DISCUSSION

Mutant pGs of VHSV with conformational changes and defective, reduced or pH-altered fusion have been obtained in the p2 phospholipid-binding region and in peptides binding to the fusion region. As the existence of VSV mutants with defective or reduced fusion that tend towards a more acid optimum pH value has previously been interpreted as an indication of the role of these mutated positions in fusion, it can be concluded that the aforementioned regions are also involved in VHSV fusion processes. Previous results achieved for penetration in membrane models by isolated p2 at the fusion pH and fusion inhibition achieved with anti-peptide antibodies corresponding to different parts (p2, frg11, p4) of the 56-144 region of the pG of VHSV (Estepa, 2001) coincide in that both peptides (p2 and peptides binding to the fusion region) are involved in any of the steps of VHSV fusion. However, as alterations have been found in pG reactivity with conformation-dependent monoclonal antibodies in all the VSVH mutants studied, it is also possible that the mutations are affecting pG conformation and that this conformational difference is responsible for the alterations in fusion that have been observed.

Despite their alteration in monoclonal antibody C10 binding, mutants P79A, L85S, R103A and T135E were capable of undergoing the low-pH conformational changes that have to precede fusion, although P79A was capable of fusion at only 50% at pH 0.3 units lower than the native pG, whilst other the mutants needed pH 5.0 (or lower) to achieve 25-50% fusion. Similarly, the VHSV mutants resistant to neutralisation by MAb C10, which had lost their capacity to bind to MAbs C10, were still capable of fusion and their mapped epitopes were linked to VHSV fusion. The VHSV mutants in which some fusion activity was maintained had mutations either flanking the innermost nucleus of p2 (P79A, L85S and R103A) or in the hydrophilic loop (p4) between the p2 and fusion peptides (T135E). In all these cases, the change in the conformation at physiological pH at positions 140 or 433 (as estimated by MAb C10 binding) and 235 (as estimated by MAb 2F1A12 binding) did not prevent fusion. The increase in the binding of MAbs C10 and 2F1A12 to the native pG at low pH could indicate that the conformation required for fusion at low pH is less affected by these mutations. Figure 1 shows that the mutants with fusion activity at positions P79 or L85 (i.e. around position 80) and T135 (i.e. around position 140) and the mutants resistant to neutralisation by MAb C10 were mapped at positions either around amino acid 80 or around amino acid 140, the two locations around which the number of amino acid changes in the 22 VHSV isolates was the highest. The locations around the amino acid variations in natural isolates of site-directed and monoclonal antibody resistant mutants that retain fusion activity (except position 103), suggest that in order to preserve fusion activity most of the amino acid changes allowed in the 56-159 region are those around positions 80 and 140.

The reduction in the binding capacity of the conformation-dependent MAbs to the mutant pG of VHSV should indicate that those mutants are poorly folded. Therefore, those mutations would be affecting conformation of the pG, which would be the main reason behind the alterations observed in fusion activity. At the moment it is not possible to determine whether the mutations studied have a direct effect on fusion due to changes in pG conformation, a direct effect on its fusion capacity, or both, as none of the VHSV mutants were recognised by MAbs C10 or 2F1A12 and no other VHSV neutralising monoclonal antibody is available yet (Fernández-Alonso et al., 1998) or any other assay to study pG conformation. Moreover, it is not yet possible to directly compare the properties of fusion-defective VSV mutants with the mutants previously described for VHSV. Therefore, alterations in the binding of conformation-dependent MAbs by VSV fusion-defective mutants have not yet been described. On the other hand, no differences were found between the wild-type VSV and fusion-defective mutants as regards the increase in pG resistance to digestion with trypsin at low pH (the biochemical assay used for conformational changes). Recognition by conformation-dependent anti-VSV MAbs could be altered in those VSV mutants, as it is known that conformation in the pG is extensively altered during fusion (Carneiro et al., 2001; Carneiro et al., 2003).

To study whether these mutations in p2 could affect fusion by reducing its phospholipid-binding properties, a series of experiments were carried out with mutated synthetic peptides corresponding to p2 sequences that show the highest PS-binding activity, as described above (Estepa et al., 1996a; Estepa et al., 1996b). To decrease PS binding in this model, it was necessary to simultaneously introduce more than three amino acid substitutions in the native p2 sequence in accordance with previous indications, in which both hydrophobic and ionic interactions were required for maximum PS binding (Estepa et al., 1996b). Although not all the possible mutations in p2 have been studied, these results made it unlikely that the mutants with a single mutation or the two double mutants studied (P86AG98A or G98AH99S) could cause a reduction in PS binding.

The pG with mutations in the hypothetical fusion peptide (F147A, P148A and W154A) were completely fusion-defective for all the pHs studied, despite being expressed in the transfected EPC cell membrane at a similar level to that of the wild type or mutants with fusion activity (Table 1). VSV mutants F125Y and P126L showed a 34% and 48% reduction in fusion, respectively, compared to that obtained with the native pG (Shokralla et al., 1999), whilst in the equivalent VHSV mutants F147K and P148K the reduction in fusion was 100%. The lower fusion activity observed in VHSV could be due to more drastic amino acid changes introduced in the VHSV mutants. Alternatively, it could be due to differences in the conditions of cell-cell fusion assays (exposure to low pH for 2 or 15 minutes in VSV or VHSV, respectively).

As the serum from rainbow trout immunised with VHSV (approximately 40%) reacted strongly with solid-phase frg11 (Estepa et al., 2001; Rocha et al., 2002) by recognising its linear epitopes (Fernández-Alonso et al., 1998) and the mutants in frg11 were expressed in the cell membrane regardless of its conformation, most of the fusion-defective pG mutants described here are viable and capable of inducing immune responses in rainbow trout. Some of the mutations described in the present invention could be used to design attenuated vaccines against VHSV, including DNA vaccines with the mutant G gene (Anderson, 1996a; Anderson, 1996b; Fernández-Alonso, 2001) or VHSV mutants obtained by reverse genetic methods (Biacchesi et al., 2002; Biacchesi et al., 2000).

**Table 1**

| Cytoplasmic (EF) and membrane (FACS) expression of pG and induced nuclei in syncytia at the optimum pH in EPC cells transfected with mutant pG | | | | | | | | | | |
|---|---|---|---|---|---|---|---|---|---|---|
| | | | **FACS** Anti-pG PAb** | | | **MAb C10⁺** | | | **Fusion** | |
| **Domain** | **Mutant** | ***IF** | **% of stained cells** | **Fluorescence intensity (rfu)** | **n** | **% of stained cells** | **n** | **pH** | **% of nuclei in syncytia ***** | **n** |
| | Wild type | + | 53.5 ± 11 | 18.7 ± 4.1 | (6) | 21.6 ± 9 | (6) | 5.6 | 44.7 ± 8.1 | (3) |
| **Upstream of p2** | P65A | + | 54.1 ± 8 | 13.3 ± 3.1 | (3) | 0.9 ± 0.1 | (4) | - | 1.8 ± 0.6 | (3) |
| | P79A | + | 42.5 ± 10 | 13.1 ± 3.6 | (4) | 0.3 ± 0.1 | (3) | 5.3 | 23.5 ± 2.4 | (2) |
| **Phospholipid-binding peptide p2 (82-110)** | I82S | + | 1.3 ± 0.3 | 0 | (3) | 1.6 ± 0.6 | (4) | ? | 1.6 ± 0.2 | (2) |
| | L85S | + | 51.6 ± 29 | 13.6 ± 1.1 | (2) | 0.5 ± 0.1 | (3) | 5.0 | 9.2 ± 3.5 | (2) |
| | P86A | + | 56.8 ± 15 | 13.7 ± 5.8 | (4) | 1.1 ± 0.6 | (3) | - | 2.1 ± 0.5 | (3) |
| | P86AG98A | + | 50.6 ± 13 | 12.2 ± 3.5 | (4) | 1.4 ± 0.1 | (4) | - | 1.4 ± 0.3 | (3) |
| | A96E | + | 44.5 ± 19 | 13.3 ± 3.3 | (3) | 1.2 ± 0.6 | (3) | - | 1.6 ± 0.4 | (2) |
| | G98A | + | 77.2 ± 9 | 13.2 ± 4.7 | (2) | 0.6 ± 0.2 | (3) | - | 1.6 ± 0.2 | (2) |
| | G98AH99S | + | 71.5 ± 4 | 22.5 ± 2.5 | (2) | 0.5 ± 0.3 | (2) | - | 1.7 ± 0.3 | (2) |
| | R103A | + | 64.7 ± 14 | 11.7 ± 3.2 | (2) | 1.6 ± 0.8 | (2) | 5.0 | 27.7 ± 4.1 | (2) |
| | R107A | + | 63.5 ± 23 | 13.1 ± 2.1 | (2) | 0.9 ± 0.3 | (2) | - | 2.1 ± 0.5 | (2) |
| **Downstream of p2** | F115K | + | 52.0 ± 23 | 10.8 ± 3.4 | (3) | 0.4 ± 0.2 | (2) | - | 1.6 ± 0.5 | (3) |
| | T135E | + | 55.6 ± 20 | 11.6 ± 8.4 | (3) | 1.3 ± 0.8 | (2) | 5.0 | 13.7 ± 4.5 | (3) |
| **Fusion peptide (142-159)** | F147K | 69.0 ± 1 | 11.5 ± 0.5 | (2) | 1.3 ± 0.3 | (3) | - | 1.6 ± 0.2 | (2) | |
| | P148K | 50.2 ± 2 | 22.5 ± 2.5 | (2) | 1.5 ± 0.3 | (2) | - | 2.8 ± 1.5 | (2) | |
| | W154K | 76.3 ± 13 | 13.1 ± 1.8 | (2) | 1.0 ± 0.7 | (3) | - | 1.8 ± 0.2 | (2) | |

| | | | | | | | | | | |
|---|---|---|---|---|---|---|---|---|---|---|
| *, Expression in the cytoplasm of EPC cells transfected with plasmids containing pG mutants was estimated by immunofluorescence with anti-pG rabbit PAbs. The results were classified into "+" when more fluorescence was detected than in the background; **, The results are given as means ± standard deviation of the percentage of EPC cells stained with the indicated antibodies, compared with the number of cells stained with the same monoclonal antibodies in non-transfected EPC cells (Figure 2). The means ± standard deviation are given for the fluorescence intensity, with the number of different experiments per mutant shown in brackets; ***, The results of fusion at the maximum pH (Figure 3) are given as the percentage of nuclei in syncytia (syncytia of 3 or more cells per syncytium, n = 1,300 approximately, 4 fields of enlargement (x100)). The percentage of nuclei in syncytia in EPC cell monolayers not transfected after pH 5.6 was 1.3 % (n=1,300), although 95% of them only had 3 nuclei per syncytium; +, Monoclonal antibody C10 maps simultaneously at positions 139 and 140 (Bearzotti, 1995; Gaudin, 1999). | | | | | | | | | | |

**Table 2**

| Labelled PS-binding of solid-phase mutant p2 (positions 93 to 107) | | |
|---|---|---|
| Position | Sequence | PS binding (Pmol /µg peptide) (Pmol /µg peptide) |
| 93 | AAVASGHYLHRVTYR | 2.17 ± 0.47 |
| 94 | SAVASGHYLHRVTYR | 2.47 ± 0.34 |
| 95 | SAAASGHYLHRVTYR | 2.10 ± 0.46 |
| 96 | SAVASGHYLHRVTYR | 2.47 ± 0.34 |
| 97 | SAVAAGHYLHRVTYR | 1.82 ± 0.50 |
| 98 | SAVASAHYLHRVTYR | 2.66 ± 0.16 |
| 99 | SAVASGAYLHRVTYR | 2.41 ± 0.15 |
| 100 | SAVASGHALHRVTYR | 2.99 ± 0.23 |
| 101 | SAVASGHYAHRVTYR | 3.24 ± 0.31 |
| 102 | SAVASGHYLARVTYR | 4.10 ± 0.53 |
| 103 | SAVASGHYLHAVTYR | 2.72 ± 0.23 |
| 104 | SAVASGHYLHRATYR | 2.80 ± 0.37 |
| 105 | SAVASGHYLHRVAYR | 2.64 ± 0.31 |
| 106 | SAVASGHYLHRVTAR | 2.84 ± 0.33 |
| 107 | SAVASGHYLHRVTYA | 2.40 ± 0.28 |
| | | |
| 107 | SAVASGHYLHRVTYK | 2.50 ± 0.42 |
| 103 | SAVASGHYLHKVTYR | 2.60 ± 0.33 |
| 103,107 | SAVASGHYLHKVTYK | 1.30 ± 0.19 |
| 103,107 | SAVASGHYLHEVTYE | 0.75 ± 0.36 |
| | | |
| 104-106 | SAVASGHYLHRAAAR | 2.10 ± 0.28 |
| 95,104-106 | SAAASGHYLHRAAAR | 1.69 ± 0.29 |
| 99-102,104-106 | SAVASGAAAARAAAR | 0.69 ± 0.21 |

Radioactively labelled PS was estimated by binding to a solid phase coated with mutant synthetic peptides deriving from the partial p2 sequence (₉₃SAVASGYLHRVTYR₁₀₇). Counts above the background value were converted to pmol of PS per µg of peptide and the averages from two different experiments were carried out in triplicate and their standard deviations are shown in the table. The amino acid sequences are shown using a letter code. The mutated amino acids are shown in bold.

### BIBLIOGRAPHY

Anderson, E.D., Mourich, D. V., Fahrenkrug, S.C., LaPatra, S.C., Shepherd, J. and Leong, J.C. (1996a). Genetic immunization of rainbow trout (Oncorhynchus mykiss) against infectious hematopietic necrosis virus. Molecular Marine Biology Biotechnology, 5: 114-122.
Anderson, E.D., Mourich, D.V., and Leong, J.C. (1996b). Gene expression in rainbow trout (Oncorhynchus mykiss) following intramuscular injection of DNA. Molecular Marine Biology Biotechnology, 5: 105-113.
Bearzotti, M., Monnier, A.F., Vende, P., Grosclaude, J., DeKinkelin, P. and Benmansour, A. (1995). The glycoprotein of viral hemorrhagic septicemia virus (VHSV): antigenicity and role in virulence. Vet.Res. 26:413-422.
Biacchesi, S., Bearzotti, M., Bouguyon, E. and Bremont, M. (2002). Heterologous exchanges of the glycoprotein and the matrix protein in a Novirhabdovirus. J.Virol. 76:2881-2889.
Biacchesi, S., Thoulouze, M.I., Bearzotti, M., Yu, Y.X. and Bremont, M. (2000). Recovery of NV Knockout Infectious Hematopoietic Necrosis Virus Expressing Foreign Genes. J.Virol. 74:11247-11253.
Carneiro, F.A., Ferradosa, A.S., y DaPoian, A.T. (2001). Low pH-induced conformational changes in vesicular stomatitis virus glycoprotein involve dramatic structure reorganization. Journal of Biological Chemistry, 276: 62-67.
Carneiro, F.A., Stauffer, F., Lima, C.S., Juliano, M.A., Juliano, L. and DaPoian, A.T. (2003). Membrane fusion induced by vesicular stomatitis virus depends on histidina protonation. Journal of Biological Chemistry, 278: 13789- 13794 .
Coll, J.M. (1995). Heptad-repeat sequences in the glycoprotein of rhabdoviruses. Virus Genes 10:107-114.
Einer-Jensen, K., Krogh, T.N., Roepstorff, P. and Lorenzen, N. (1998). Characterization of intramolecular disulphide bonds and secondary modifications of the glycoprotein from viral haemorrhagic septicaemia virus (VHSV), a fish rhabdovirus. J.Virol. 72:10189-10196.
Estepa, A. and Coll, J.M. (1996a). Pepscan mapping and fusion related properties of the major phosphatidylserine-binding domain of the glycoprotein of viral haemorrhagic septicaemia virus, a salmonid rhabdovirus. Virology 216:60-70.
Estepa, A. and Coll, J.M. (1996b). Phosphatidylserine binding to solid-phase peptides: a new method to study phospholipid/viral protein interactions. J.Virol. Methods 61 :37-45.
Estepa, A., Rocha, A., pérez, L., Encinar, J.A., Núñez, E., Fernández, A., González Ros, J.M., Gavilanes, F. and Coll, J.M. 2001. A protein fragment from the salmonid VHS rhabdovirus induces cell-to-cell fusion and membrane phosphatidylserine translocation at low pH. Journal Biological Chemistry, 276:46268-46275.
Fernández-Alonso, M., Alvarez, F., Estepa, A., Blasco, R. and Coll, J.M. (1999). A model to study fish DNA immersion- vaccination by using the green fluorescent protein. J.Fish Dis. 22:237-241.
Fernández-Alonso, M., Lorenzo, G., Pérez, L., Bullido, R., Estepa, A., Lorenzen, N. and Coll, J.M. (1998). Mapping of the lineal antibody epitopes of the glycoprotein of VHSV, a salmonid rhabdovirus. Diseases Aquatic Organisms, 34: 167- 176
Fernández-Alonso, M., Rocha, A. and Coll, J.M. (2001). DNA vaccination by immersion and ultrasound to trout viral haemorrhagic septicaemia virus. Vaccine, 19: 3067-3075 .
Fijan, N.; Sulimanovic, D.; Bearzotti, M.; Muzinic, D.; Zwillenberg, L.O.Z.; Chümonczyk, S; Vautherot, J.F. and Kinkelin, P. (1983). Some properties of the epithelioma papulosum cyprinid (EPC) cell line from carp cyprinus carpio. Annals Virology (Institute Pasteur), 134: 207-220.
Fredericksen, B.L., and Whitt, M.A. (1996). Mutations al two conserved acidic amino acids in the glycoprotein of vesicular stomatitis virus affect pH-dependent conformational changes and reduce the pH threshold for membrane fusion. Virology, 217: 49-57.
Gaudin, Y., DeKinkelin, P. and Benmansour, A. (1999). Mutations in the glycoprotein of viral haemorrhagic septicaemia virus that affect virulence for fish and the pH threshold for membrane fusion. J.Gen.Virol. 80:1221-1229.
Gaudin, Y., Ruigrok, R.W.H., Knowssow, M. and Flamand, A. (1993). Low-pH conformational changes of rabies virus glycoprotein and their role in membrane fusion. Journal Virology, 67: 1365-1372.
Heike, S., Egbert, M. and Mettenleiter, T.C. (1999). Complete genomic sequence of viral haemorrhagic septicaemia virus, a fish rhabdovirus. Virus Genes 19:59-65.
López, A., Fernández-Alonso, M., Rocha, A., Estepa, A. and Coll, J.M. (2001). Transfection of epithelioma cyprini (EPC) carp cells. Biotechnology Letters 23:481-487.
Lorenzen, N. and LaPatra, S.E. (1999). Immunity to rhabdoviruses in rainbow trout: the antibody response. Fish Shellfish Immunology, 9: 345-360.
Rocha, A., Fernández-Alonso, M., Mas, V., Pérez, L., Estepa, A. and Coll, J.M. (2002). Antibody response to a linear epitope of the protein G of a rhabdovirus in immunized trout. Vet.Immunol.Immunopathol. 86:89-99 .
Rocha, A., Ruiz, S., and Coll, J.M. (2004a). Improvement of transfection efficiency of epithelioma papulosum cyprini carp cells by modification of their cell cycle and using an optimal promoter. Marine Biotechnology, in press.
Rocha, A., Ruiz, S., Tafalla, C. and Coll, J.M. (2004b). Characterisation of the syncyntia formed by VHS salmonid rhabdovirus G-gene transfected cells. Veterinary Immunology Immunopathology, in press.
Shokralla, S., Chernish, R. and Ghosh, H.P. (1999). Effects of double-site mutations of vesicular stomatitis virus glycoprotein G on membrane fusion activity. Virology 256:119-129.
Thiry, M.; Lecoq-Xhonneux, F.; Dheur, L; Renard, A. and Kinkelin, D. (1991). Molecular cloning of the m_RNA encoding for the G protein of the viral haemorrhagic septicaemia (VHS) of salmonids. Journal Veterinary Microbiology, 23: 221- 226.
Walker, P. J. and Kongsuwan, K. (1999). Deduced structural model for animal rhabdovirus glycoproteins. J.Gen.Virol. 80:1211-1220.

## Claims

1. A mutant G gene of viral haemorrhagic septicaemia virus (VHSV), which encodes a mutant G protein (pG) of VHSV, wherein said mutant pG of VHSV comprises at least one mutation with defective or null binding to cells that can be infected by VHSV.

2. Mutant G gene according to claim 1, which encodes a mutant pG of VHSV whose mutation is located upstream of the p2 domain of the pG of VHSV.

3. Mutant G gene according to claim 2, wherein said mutation in the pG of VHSV is selected from mutations P65A and P79A.

4. Mutant G gene according to claim 1, which encodes a mutant pG of VHSV whose mutation is located in the phospholipid (p2) binding domain of the pG of VHSV.

5. Mutant G gene according to claim 4, wherein said mutation in the pG of VHSV is selected from mutations I82S, L85S, P86A, P86AG98A, A96E, G98A, G98AH99S, R103A and R107A.

6. Mutant G gene according to claim 1, which encodes a mutant pG of VHSV whose mutation is located downstream of the p2 domain of the pG of VHSV.

7. Mutant G gene according to claim 6, wherein said mutation in the pG of VHSV is selected from mutations F115k and T135E.

8. Mutant G gene according to claim 1, which encodes a mutant pG of VHSV whose mutation is located in the fusion peptide domain of the pG of VHSV.

9. Mutant G gene according to claim 8, wherein said mutation in the pG of VHSV is selected from mutations F147K, P148K and W154K.

10. A vector comprising a mutant G gene according to any of claims 1 to 9.

11. Vector according to claim 10, selected from a DNA plasmid and an expression vector that can be expressed in eukaryotic cells.

12. Vector according to claims 10 or 11, which also comprises the necessary elements for the expression and translation of said mutant G gene and/or elements that regulate its transcription and/or translation.

13. Use of a vector according to any of claims 10 to 12 to produce a vaccine for protecting animals that can be infected by VHSV.

14. A mutant viral haemorrhagic septicaemia virus (VHSV) whose genome comprises a mutant G gene according to any of claims 1 to 9, and the other VHSV genes.

15. Use of a mutant viral haemorrhagic septicaemia virus (VHSV) according to claim 14 to produce a vaccine for protecting animals that can be infected by VHSV.

16. A vaccine that comprises a mutant G gene according to any of claims 1 to 9, and, optionally, one or more adjuvants and/or pharmaceutically acceptable vehicles.

17. Vaccine according to claim 16, wherein said mutant G gene is incorporated into a vector according to any of claims 10 to 12.

18. Vaccine according to claim 16, wherein said mutant G gene is incorporated into a mutant VHSV according to claim 14.

19. Vaccine according to claim 16, selected from a DNA vaccine and an attenuated live vaccine.

20. Vaccine according to any of claims 16 to 19, for protecting animals that can be infected by VHSV.

21. Vaccine according to claim 20, wherein said animals that can be infected by VHSV are aquatic animals.

22. Vaccine according to claim 21, wherein said aquatic animals are selected from salmonids, cod, turbot, croaker, eels, John Dory and prawns.

23. Vaccine according to claims 21 or 22, wherein said aquatic animals are rainbow trout.

24. A transgenic non-human animal whose cells contain a gene G mutant according to any of claims 1 to 9 integrated into their genome.

25. Animal according to claim 24, **characterised in that** it is a fish.

26. A mutant G protein of viral haemorrhagic septicaemia virus (VHSV) encoded by a mutant G gene according to any of claims 1 to 9.

27. Mutant G protein according to claim 26, selected from a mutant pG with the P65A mutation; a mutant pG with the P79A mutation; a mutant pG with the I82S mutation; a mutant pG with the L85S mutation; a mutant pG with the P86A mutation; a mutant pG with the P86AG98A mutation; a mutant pG with the A96E mutation; a mutant pG with the G98A mutation; a mutant pG with the G98AH99S mutation; a mutant pG with the R103A mutation; a mutant pG with the R107A mutation; a mutant pG with the F11K mutation; a mutant pG with the T135E mutation; a mutant pG with the F147K mutation; a mutant pG with the P148K mutation; and a mutant pG with the W154K mutation.

28. A host cell comprising a mutant G gene according to any of claims 1 to 9.

29. Host cell according to claim 28, **characterised in that** it is a eukaryotic cell.

30. Host cell according to claim 29, **characterised in that** it is a cell belonging to the EPC (Epithelioma Papullosum cyprisi) cell line.

31. A method for producing a mutant G protein of viral haemorrhagic septicaemia virus (VHSV) that consists of culturing a host cell according to either of claims 28 or 29, which comprises a mutant G gene according to any of claims 1 to 9, under conditions that allow the expression of said gene.

32. Method according to claim 31, which also consists of removing the mutant G protein produced from the culture medium.
